# EUROPEAN PATENT APPLICATION

(11) **EP 4 778 910 A1**
(43) Date of publication of application: **22.07.2026**
(21) Application number: 23952131.3
(22) Date of filing: 28.12.2023
(51) Int. Cl.: C07C 15/073, B01J 29/40, C01B 39/38

(54) **ZSM-5 MOLECULAR SIEVE, CATALYST FOR PREPARING ETHYLBENZENE BY MEANS OF ALKYLATION OF BENZENE, AND PREPARATION AND USE THEREOF**

(30) Priority: 12.09.2023 CN 202311174098
(71) Applicant: CHINA PETROLEUM & CHEMICAL CORPORATION, Chaoyang District Beijing 100728 (CN); Sinopec (Shanghai) Research Institute of Petrochemical Technology Co., Ltd., Shanghai 201208 (CN)
(72) Inventor: YANG, Weimin, Shanghai 201208 (CN); WANG, Darui, Shanghai 201208 (CN); SUN, Hongmin, Shanghai 201208 (CN); HUAN, Mingyao, Shanghai 201208 (CN); LIU, Wei, Shanghai 201208 (CN); WANG, Yiyan, Shanghai 201208 (CN)
(74) Representative: Hoffmann Eitle
(86) International application number: PCT/CN2023/142629
(87) International publication number: WO 2025/055213

(57) **Abstract**

A ZSM-5 molecular sieve, a catalyst for preparing ethylbenzene by means of the alkylation of benzene, and the preparation and use thereof. The ZSM-5 molecular sieve does not have twin crystals; the dimension (La) in the a-axis direction is equal to 240-300 nm, the dimension (Lb) in the b-axis direction is equal to 60-90 nm, and the dimension (Lc) in the c-axis direction is equal to 1500-2200 nm; the dimension (Ld) of a crystal edge formed by the intersection of (010) and (101) crystal faces is equal to 180-220 nm, and the dimension (Le) of a crystal edge formed by the intersection of (010) and (100) crystal faces is equal to 1300-1900 nm; and the ratio (La/Lb) of the dimension in the a-axis direction to the dimension in the b-axis direction is equal to 2.5-4.5, and the ratio (Lc/Lb) of the dimension in the c-axis direction to the dimension in the b-axis direction is equal to 22.0-26.0. When the catalyst for preparing ethylbenzene by means of the alkylation of benzene, which catalyst is prepared from the ZSM-5 molecular sieve, is used in a reaction for ethylbenzene preparation by means of benzene alkylation, the conversion rate of ethylene is high, the selectivity of ethyl is high in an alkylation product, and the mass content of the critical impurity xylene is low.

## Description

### Technical Field

The invention belongs to the technical fields of catalytic chemistry and chemical engineering, and in particular relates to a ZSM-5 molecular sieve, a benzene alkylation catalyst, and a preparation method and application thereof.

### Background Art

Benzene alkylation products such as ethylbenzene are important basic organic raw materials, which can be used in many fields and play an important role in the development of the national economy.

The technology of alkylating benzene with ethylene to produce ethylbenzene has a wide range of sources of raw materials and, depending on reaction processes, can be divided into liquid-phase method and gas-phase method. The gas-phase method is suitable for various ethylene feeds, which can be pure ethylene, dilute ethylene, ethanol, or the like, having a mass concentration of ethylene of 10 to 100 wt.%. The dilute ethylene feeds are mainly derived from tail gases of FCC and DCC devices or crude ethane cracking gases and are cheaper than polymerization grade pure ethylene.

The cheap, dilute ethylene feeds contain many impurities and may cause serious side reactions during the reaction, resulting in reduced selectivity of ethyl products. The reaction temperature of gas-phase processes is high, generally exceeding 300 °C, and the alkylation of benzene and ethylene is a highly exothermic reaction, which leads to a significant temperature rise inside the alkylation reactor, thereby increasing the probability of side reactions and the rate of coking so that the content of key impurities, i.e., xylenes, in the ethylbenzene product increases significantly and the service life of the catalyst is greatly shortened. The key for the technology of producing ethylbenzene via gas-phase alkylating benzene with ethylene is to develop an alkylation catalyst with high activity, high selectivity and high stability and suitable for various ethylene feeds.

The technology of producing ethylbenzene via gas-phase alkylating benzene with ethylene has been extensively researched, and the active component of the catalysts for gas-phase alkylating benzene with ethylene to produce ethylbenzene is generally ZSM-5 molecular sieve.

Chinese patent application CN102875316A discloses a method for producing ethylbenzene by alkylation of dry gas and benzene, which method uses dry gas and benzene as reaction raw materials and comprises contacting the reaction raw materials with a catalyst to conduct a gas-phase alkylation to generate ethylbenzene, wherein the catalyst comprises the following components: a) 40 to 90 wt% of a ZSM-5 molecular sieve with a grain diameter of 5 to 500 nm and a SiO₂/Al₂O₃ molar ratio of 30 to 400; b) 9 to 59 wt% of alumina or silica as a binder; and c) 0.1 to 10 wt% of an alkaline earth metal oxide and 0.1 to 10 wt% of a rare-earth metal oxide. A technical solution where the catalyst is treated with a high-temperature steam solves well the problems of poor catalyst stability and short regeneration cycle, but the feed ethylene applicable to the method is dry gas, in which the ethylene mass concentration is generally 10 to 20 wt%, and it is not further specified whether a feed gas with a higher ethylene concentration is applicable to the method.

Chinese patent application CN101993331A discloses a method for producing ethylbenzene by alkylating benzene with pure ethylene or dry gas. The method uses a fixed bed reactor loaded with at least one section of ZSM-5 molecular sieve catalyst I with a SiO₂/Al₂O₃ molar ratio of 50-150 and at least one section of ZSM-5 molecular sieve catalyst II with a SiO₂/Al₂O₃ molar ratio of 160-300. 30%-70% of the pure ethylene or dry gas enters the bed layer loaded with the catalyst I, and the remaining pure ethylene or dry gas enters the bed layer loaded with the catalyst II; 80%-100% of benzene enters a first bed layers, where the catalyst I is loaded, from the top of the reactor, and the remaining benzene enters the subsequent catalyst bed layers sectionwise, so as to control the difference between the inlet temperature of each catalyst bed layer and the inlet temperature at the top of the reactor to not exceed ±5°C.

Chinese patent application CN108080019A discloses a method for preparing a high-selectivity benzene alkylation strip-shaped catalyst, which method utilizes a MFI zeolite molecular sieve with a high silica-alumina ratio as a catalyst precursor and comprises the steps of: subjecting a mixture of a hydrolysate of acidified silicone grease and the MFI zeolite molecular sieve with the high silica-alumina ratio to strip-extrusion molding; treating so-formed catalyst with a mother liquor separated during the synthesis of the MFI zeolite molecular sieve with the high silica-alumina ratio through hydrothermal crystallization, with a boride being added into the separated mother liquor; and converting the mother liquor-treated strip extrudes of the MFI zeolite molecular sieve with the high silica-alumina ratio via ion exchange into a H-type alkylation catalyst. Said method utilizes the mother liquor obtained during the hydrothermal synthesis of the MFI zeolite molecular sieve with the high silica-alumina ratio to treat the molded strip-shaped catalyst, thereby not only recovering the useful components in the mother liquor and dredging the pores of the catalyst but also reducing the environmental pollution in the entire production chain of the alkylation catalyst. The mechanical strength of catalyst particles is improved by adding an appropriate amount of boride into the mother liquor when the molded catalyst is treated with the mother liquor, and this is beneficial to prolonging the service life of the catalyst. However, this method does not compare in detail the specific lifespan of the catalyst, and the applicable raw materials are ethanol or methanol, without further explanation as to whether the method is applicable to dilute ethylene or pure ethylene feeds.

Other documents related to the background art include CN111111748A, CN114733560A, CN103073020A, and CN107512729A.

Therefore, there is still a need to provide benzene alkylation catalysts that exhibit desirable properties.

### Disclosure of the invention

The inventors have found through diligent research that morphologies, axial dimensions and ratios therebetween, and crystal face areas and ratios therebetween of ZSM-5 molecular sieves have an important influence on the performance of the benzene alkylation catalysts. The inventors have found through further research that, in the process of preparing the molecular sieves, by introducing an organic matter II and an organic matter III at different times, respectively, the morphology, axial dimensions and ratios therebetween of the sheet-like molecular sieves can be effectively controlled, and the areas of the three crystal faces (100), (010) and (101) and ratios therebetween are also within specific ranges. So-prepared ZSM-5 molecular sieve is a novel ZSM-5 molecular sieve, and its use in a benzene alkylation catalyst for producing ethylbenzene can enhance the ethylene conversion rate, improve the selectivity to the ethyl product in the alkylation products, inhibit side reactions, reduce the content of xylenes, and prolong the service life of the catalyst. Thus, the present invention is made.

In a first aspect, the present invention provides a ZSM-5 molecular sieve, wherein there are no twin crystals in the molecular sieve, and the molecular sieve has a size in the a-axis direction, La, of 240 to 300 nm, for example, 250 nm, 260 nm, 270 nm, 280 nm, or 290 nm, or in a range between any two of these values; a size in the b-axis direction, Lb, of 60 to 90 nm, for example, 65 nm, 70 nm, 75 nm, 80 nm, or 85 nm, or in a range between any two of these values; and a size in the c-axis direction, Lc, of 1,500 to 2,200 nm, for example, 1,550 nm, 1,600 nm, 1,650 nm, 1,700 nm, 1,750 nm, 1,800 nm, 1,850 nm, 1,900 nm, 1,950 nm, 2,000 nm, 2,050 nm, 2,100 nm, or 2,150 nm, or in a range between any two of these values. For the molecular sieve, a size of a crystal edge formed by the intersection of (010) and (101) crystal faces, Ld, is from 180 to 220 nm, for example, 185 nm, 190 nm, 195 nm, 200 nm, 205 nm, 210 nm or 215 nm, or in a range between any two of these values; a size of a crystal edge formed by the intersection of (010) and (100) crystal faces, Le, is from 1,300 to 1,900 nm, for example, 1,350 nm, 1,400 nm, 1,450 nm, 1,500 nm, 1,550 nm, 1,600 nm, 1,650 nm, 1,700 nm, 1,750 nm, 1,800 nm or 1,850 nm, or in a range between any two of these values; a ratio of the a-axis direction size to the b-axis direction size, La/Lb, is from 2.5 to 4.5, for example, 2.7, 2.9, 3.1, 3.3, 3.5, 3.7, 3.9, 4.1 or 4.3, or in a range between any two of these values; and a ratio of the c-axis direction size to the b-axis direction size, Lc/Lb, is from 22.0 to 26.0, for example, 22.5, 23.0, 23.5, 24.0, 24.5, 25.0 or 25.5, or in a range between any two of these values.

In some preferred embodiments, for the molecular sieve, an area of the (100) crystal face, S1, is from 75,000 to 180,000 nm², for example, 80,000, 90,000, 100,000, 110,000, 120,000, 130,000, 140,000, 150,000, 160,000 or 170,000 nm², or in a range between any two of these values; an area of the (010) crystal face, S2, is from 350,000 to 600,000 nm², for example, 400,000, 450,000, 500,000 or 550,000 nm², or in a range between any two of these values; an area of the (101) crystal face, S3, is from 10,000 to 20,000 nm², for example, 11,000, 12,000, 13,000, 14,000, 15,000, 16,000, 17,000, 18,000 or 19,000 nm², or in a range between any two of these values; a ratio of the (100) crystal face area to the (010) crystal face area, S1/S2, is from 0.20 to 0.30, for example, 0.21, 0.22, 0.23, 0.24, 0.25, 0.26, 0.27, 0.28 or 0.29, or in a range between any two of these values; and a ratio of the (101) crystal face area to the (010) crystal face area, S3/S2, is from 0.025 to 0.045, for example, 0.027, 0.029, 0.031, 0.033, 0.035, 0.037, 0.039, 0.041 or 0.043. Preferably, in the molecular sieve, an exposure degree of the (010) crystal face, defined as S2/(S1+S2+2S3)×100%, is from 70 to 80 %, for example, 71%, 72%, 73%, 74%, 75%, 76%, 77%, 78% or 79%.

In some preferred embodiments, the infrared spectrum of the molecular sieve has two peaks P1 and P2 in the wavenumber range of 690-750 cm⁻¹, with the vertexes of the peaks P1 and P2 falling within wavenumber ranges of 690-710 cm⁻¹ and 730-750 cm⁻¹, respectively, and two peaks P3 and P4 in the wavenumber range of 440-560 cm⁻¹, with the vertexes of the peaks P3 and P4 falling within wavenumber ranges of 440-460 cm⁻¹ and 540-560 cm⁻¹, respectively.

In some embodiments, the ZSM-5 molecular sieve has a SiO₂:Al₂O₃ molar ratio of 100-300.

In some embodiments, the molecular sieve in an as-synthesized state has a schematic chemical composition, in moles, including: SiO₂:nAl₂O₃:mE2:xE3, where E2 represents an organic matter II, E3 represents an organic matter III, n is a value of 0.0033 to 0.01, m is a value of 0.0015 to 0.005, and x is a value of 0.005 to 0.1. The as-synthesized state of the molecular sieve refers to an uncalcined state after synthesis. When the molecular sieve exists in the as-synthesized state, it may further contain a template and water. The template may be any of ones known in the art to give a ZSM-5 molecular sieve, such as one or more of tetrapropylammonium bromide, tetrapropylammonium hydroxide, ethylamine, ethylenediamine, n-propylamine, propylenediamine, n-butylamine, tetramethylenediamine, n-hexylamine, hexamethylenediamine, ammonia, hexamethyleneimine, piperidine, and piperazine. The molar ratio of the template to SiO₂ can be from 0.05:1 to 0.3: 1. The molar ratio of water to SiO₂ can be from 3:1 to 20:1.

According to the present disclosure, the organic matter II has a structural formula 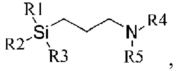 wherein R1-R3 are each independently selected from the group consisting of C1-C6 alkoxy and C1-C6 alkoxy-C1-C6 alkyl, and preferably methoxy, ethoxy or 3-methoxypropyl, and R4 and R5 are each independently selected from the group consisting of hydrogen, C1-C6 alkyl and amino-C1-C6 alkyl, and preferably hydrogen, methyl, ethyl, aminomethyl or aminoethyl. Examples include, but are not limited to, 3-aminopropyl methoxy diethoxy silane, 3-aminopropyl triethoxy silane, and aminoethyl aminopropyl di(methoxyethoxy) silane.

According to the present disclosure, the organic matter III has a structural formula 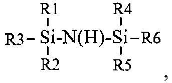 wherein R1-R6 are each independently selected from the group consisting of methyl, ethyl, propyl and phenyl, provided that one, two, three or four of R1-R6 are phenyl.

In a second aspect, the present invention provides a method for preparing the ZSM-5 molecular sieve, comprising:
(a) mixing a silicon source I, an optional aluminum source I, water, an organic matter I and an organic matter II to obtain a mixture A;
(b) treating the mixture A to obtain a mixture B having a morphology-directing effect;
(c) mixing the mixture B, a silicon source II, an aluminum source II, water, a template and an organic matter III to obtain a mixture C; and
(d) allowing the mixture C to crystallize, followed by liquid-solid separation, washing and drying, to obtain the molecular sieve.

In some embodiments, step (a) has preferably one or more of the following features:
- the silicon source I in step (a) is one or more of tetramethyl orthosilicate, tetraethyl orthosilicate, tetrapropyl orthosilicate, tetrabutyl orthosilicate and tetraphenyl orthosilicate;
- the aluminum source I in step (a) is one or more of aluminum sulfate, aluminum isopropoxide, sodium aluminate, and pseudo-boehmite;
- the organic matter I in step (a) is one or more of tetrapropylammonium hydroxide, ethylamine, ethylenediamine, n-butylamine, tetramethylendiamine, hexamethyleneimine, and piperidine;
- the organic matter II in step (a) has a structural formula 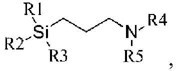 wherein R1-R3 are each independently selected from the group consisting of C1-C6 alkoxy and C1-C6 alkoxy-C1-C6 alkyl, and preferably methoxy, ethoxy or 3-methoxypropyl, and R4 and R5 are each independently selected from the group consisting of hydrogen, C1-C6 alkyl and amino-C1-C6 alkyl, and preferably hydrogen, methyl, ethyl, aminomethyl or aminoethyl; and
- in step (a), a molar ratio of the silicon source I in terms of SiO₂ to the aluminum source I in terms of Al₂O₃ to the water to the organic matter I to the organic matter II is 1 : (0-0.02) : (5-20) : (0.1-1) : (0.05-0.3).

In some embodiments, the treating in step (b) is preferably carried out at 55-85 °C under closed conditions with stirring for 6-24 hours.

In some embodiments, step (c) has preferably one or more of the following features:
- the silicon source II in step (c) is one or more of silica sol, white carbon black, silicon powder, tetramethyl orthosilicate, tetraethyl orthosilicate, tetrapropyl orthosilicate, tetrabutyl orthosilicate and tetraphenyl orthosilicate;
- the aluminum source II in step (c) is one or more of aluminum nitrate, aluminum chloride, aluminum sulfate, aluminum isopropoxide, sodium aluminate, and pseudo-boehmite;
- the template in step (c) is one or more of tetrapropylammonium bromide, tetrapropylammonium hydroxide, ethylamine, ethylenediamine, n-propylamine, propylenediamine, n-butylamine, butylenediamine, n-hexylamine, hexamethylenediamine, ammonia water, hexamethyleneimine, piperidine, and piperazine;
- the organic matter III in step (c) has a structural formula 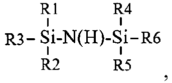 wherein R1-R6 are each independently selected from the group consisting of methyl, ethyl, propyl and phenyl, provided that one, two, three or four of R1-R6 are phenyl; and
- in step (c), a molar ratio of the silicon source II in terms of SiO₂ to the aluminum source II in terms of Al₂O₃ to the water to the template to the organic matter III is 1 : (0.0033-0.01) : (8-20) : (0.1-0.3) : (0.01-0.10), and a ratio of the mass of the mixture B to the total mass of the five substances including silicon source II, aluminum source II, water, template and organic matter III is (0.05-0.2) : 1.

In some embodiments, step (d) has preferably one or more of the following features:
- in step (d), the crystallizing is carried out by crystallizing under closed conditions at 110-170 °C for 12-72 hours; and
- in step (d), the liquid-solid separation is filter paper filtration, centrifugation, plate-frame filter press, or the like; the washing is to wash solids obtained by liquid-solid separation with deionized water multiple times until the pH value of the washing liquid is 7-8; and the drying is to dry at 100-200 °C for 5-12 hours.

Without being bound by any theory, it is believed that in the method of the present invention, the use of the organic matter II enables the mixture A to give small-sized nano-crystals modified with organic groups, and the use of the organic matter III enables the mixture C to give phenyl-modified plate-like crystals with a relatively short b-axis size and a relatively long c-axis size.

In a third aspect, the present invention provides a catalyst for producing ethylbenzene by alkylation of benzene, wherein the catalyst comprises a ZSM-5 molecular sieve, of which an as-synthesized state is the molecular sieve provided in the first aspect or the molecular sieve prepared by the method of the second aspect.

In some embodiments, the ZSM-5 molecular sieve is present in a calcined state in the catalyst.

In some embodiments, the catalyst may optionally include a binder, such as alumina, silica, or a mixture thereof, in addition to the ZSM-5 molecular sieve.

In some embodiments, preferably, the content of the molecular sieve in the catalyst is 80 mass % or more, and preferably from 80 mass % to 90 mass %, and the content of the binder in the catalyst is 20 mass % or less, and preferably from 10 mass % to 20 mass %, based on the total mass of the catalyst.

In a fourth aspect, the present invention provides a method for preparing the catalyst for producing ethylbenzene by alkylation of benzene, comprising:
(e) mixing at least one of the molecular sieves as described in the first aspect and the molecular sieves prepared in the second aspect, a binder and a polymer mixture, and then molding the resultant mixture, followed by drying, calcining, ammonium exchanging, steam treating, and acid washing, to obtain the catalyst.

In some embodiments, step (e) has preferably one or more of the following features:
- the binder is alumina, silica or a mixture thereof;
- the polymer mixture is a mixture of starch and a surfactant, with the starch accounting for 55 % to 70 % by mass of the polymer mixture; the surfactant can be selected from, for example, cationic surfactants and non-ionic surfactants, such as at least one of octadecyltrimethylammonium chloride, hexadecyltrimethylammonium bromide, P123 (available from BASF, Germany), F127 (available from BASF, Germany), polyethylene glycol ether or polyoxyethylene ethyl ether;
- a mass ratio of the molecular sieve E to the binder to the polymer mixture is 1 : (0.1-0.4) : (0.03-0.2); and
- at least one molding aid may be added in step (e), such as a peptizing acid (such as a dilute nitric acid, for example, an aqueous solution of nitric acid with a mass concentration of 3% to 10%), an extrusion aid (such as sesbania powder), and the like.

In some embodiments, in step (e), a mass ratio of the molecular sieve E to the dilute nitric acid to the sesbania powder is preferably 1 : (0.5-1.2) : (0.01-0.05).

In some embodiments, the molding in step (e) is preferably extrusion molding.

In some embodiments, the molded shape in step (e) is preferably cylindrical shape, three-lobed shape, four-lobed shape, gear shape, or the like.

In some embodiments, conditions for the drying include a temperature of 100-200 °C and a period of time of 5-12 hours; conditions for the calcining include a temperature of 500-600 °C and a period of time of 4-10 hours; conditions for the ammonium exchanging include an ammonium salt solution concentration of 2-10 % by mass, a temperature of 20-80 °C and a period of time of 1-10 hours, with the exchanging being conducted 2-5 times and the ammonium salt being at least one of ammonium sulfate, ammonium chloride, ammonium acetate and ammonium nitrate; conditions for the steam treating include a saturated water vapor atmosphere, a temperature of 300-450 °C and a period of time of 4-10 hours; and conditions for the acid washing include an acid solution concentration of 0.1-3.0 % by mass, a temperature of 20-80 °C and a period of time of 1-10 hours, with the acid being at least one of hydrochloric acid, sulfuric acid, nitric acid and oxalic acid.

In a fifth aspect, the present invention provides a catalyst for producing ethylbenzene by alkylation of benzene, which is prepared by the method of the fourth aspect.

In some embodiments, a content of the molecular sieve in the catalyst is preferably at least 80 mass %, and more preferably from 80 to 90 mass %, based on the mass of the catalyst, and a content of the binder in the catalyst is preferably at most 20 mass %, and more preferably from 10 to 20 mass %, based on the mass of the catalyst.

In a sixth aspect, the present invention provides a method for producing ethylbenzene by alkylation of benzene, comprising contacting the reaction raw materials, benzene and ethylene, with the above-described catalyst to produce a reaction effluent comprising ethylbenzene.

In some embodiments, the feed ethylene has preferably an ethylene mass concentration of 10 to 100%.

In some embodiments, preferably, the reaction temperature is 320-380 °C, the reaction pressure is 0.5-2.6 MPa, the ethylene mass space velocity is 0.2-2.5 h⁻¹, and the molar ratio of benzene to ethylene is 4.0-7.0.

Compared with the prior art, the present invention has achieved the following beneficial effects:
1. The inventors have found through research that morphologies, axial dimensions and ratios therebetween, and crystal face areas and ratios therebetween of ZSM-5 molecular sieves have an important influence on the performance of benzene alkylation catalysts. Since the crystal growth process of ZSM-5 molecular sieves follows the Ostwald-ripening and Wulff Construction rules, most of ZSM-5 molecular sieves prepared by conventional methods are of coffin morphology, and special sheet-like morphologies, specific axial dimensions and ratios therebetween, and specific crystal face areas and ratios therebetween are difficult to control. The inventors have found through further research that, in the process of preparing the molecular sieves, by introducing the organic matter II and the organic matter III at different times, respectively, the sheet-like morphology of the molecular sieves can be effectively controlled, the axial dimensions and ratios therebetween are within specific ranges, and the areas of the three crystal faces (100), (010) and (101) and ratios therebetween are also within specific ranges. The use of the ZSM-5 molecular sieve of the present invention in the benzene alkylation for producing ethylbenzene can significantly help to improve the ethylene conversion rate, inhibit side reactions, reduce the content of key impurities (i.e., xylenes), and prolong the service life of the catalyst.
2. The ZSM-5 molecular sieve of the present invention contains phenyl groups, and its infrared spectrum shows two peaks in a wavenumber range of 690-750 cm⁻¹. It is believed that using the molecular sieve of the present invention can effectively inhibit the condensation of the binder and the silanol groups on the surface of the molecular sieve during the molding process, reduce the pore blocking effect of the binder, and help to improve the comprehensive performance of the catalyst. After molding and calcining, the molecular sieve reserves on its surface a space equivalent to the size of the phenyl group, which is conducive to the enrichment of benzene and in turn increases the local benzene-to-ethylene ratio, thereby helping to reduce the benzene-to-ethylene ratio in the total feed and in turn to save energy and reduce consumption during the reaction process.
3. In the preparation of the catalyst of the present invention, not only a specific molecular sieve is used, but also a polymer mixture is added during the molding process so that the blockage of the pores of the molecular sieve by the binder is reduced. The resulting catalyst has a higher pore volume, a suitable mechanical strength and a suitable bulk density, and can improve the diffusion behavior of the reactant molecules during the reaction process, which is beneficial to improving the ethylene conversion rate, inhibiting side reactions, reducing the content of xylenes, and prolonging the life of the catalyst. The catalyst of the present invention is particularly suitable for the gas-phase alkylation of industrial benzene and ethylene to produce ethylbenzene and is applicable to a variety of ethylene feeds, including pure ethylene, dilute ethylene and ethanol.
4. Since the alkylation of benzene and ethylene is a highly exothermic reaction, the temperature rise inside the alkylation reactor will be significant, the probability of side reactions and the rate of coking will be increased so that the content of key impurities, i.e., xylenes, in the ethylbenzene product will be increased significantly and the service life of the catalyst will be greatly shortened. The catalyst of the present invention is particularly suitable for the gas-phase alkylation of benzene and ethylene to produce ethylbenzene. Under the reaction conditions of a low molar ratio of benzene to ethylene, the ethylene conversion rate is close to 100%, the selectivity of ethyl product among the alkylation products is greater than 99.6%, and the mass content of xylenes, key impurities, is less than 600 ppm.
5. The method of the present invention can use any ethylene feed, effectively expand the source of ethylene feed and alleviate the tension of ethylene feed supply, thereby leading to more economic benefits.

### Brief description of the drawings

FIG. 1 is an XRD pattern of the molecular sieve prepared in the inventive Example 1;
FIG. 2 is an SEM photograph of the molecular sieve prepared in the inventive Example 1;
FIG. 3 is an infrared spectrum of the molecular sieve prepared in the inventive Example 1;
FIG. 4 is an XRD pattern of the molecular sieve prepared in the Comparative Example 1; and
FIG. 5 is an SEM photograph of the molecular sieve prepared in the Comparative Example 1.

### Examples

The present invention will be described in detail in conjunction with specific examples below, but it should be understood that the protection scope of the present invention is not limited to these examples.

In the following examples and comparative examples, an S-4800 cold field emission high-resolution scanning electron microscope (available from Hitachi Co., Japan) was used to acquire SEM photos of the molecular sieves, and the axial sizes and crystal edge sizes of the molecular sieves were obtained by statistically analyzing data of at least 300 grains in the SEM photos. The axis designations and crystal face names of the ZSM-5 molecular sieves are shown in the following figure:

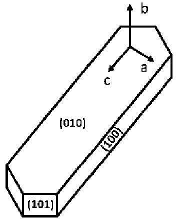

The area S1 of the (100) crystal face is Lb×Le, the area S2 of the (010) crystal face is La×Le+La×(Lc-Le)/2, the area S3 of the (101) crystal face is Lb×Ld, and the exposure degree of the (010) crystal face is S2/(S1+S2+2S3)×100%.

In the following examples and comparative examples, XRD patterns of the molecular sieves were acquired by using an Ultima IV X-ray powder diffractometer (available from Rigaku Corporation, Japan), acquiring conditions including a voltage of 35 kV, a current of 30 mA, and a scanning speed of 1°/min.

In the following examples and comparative examples, SiO₂/Al₂O₃ molar ratios of the molecular sieves were determined by using a Model S-35 ICP-AES analyzer (available from Kontron Co., Germany). Prior to the measurement, 50 mg of a sample was completely dissolved in 50 g of hydrofluoric acid solution.

In the following examples and comparative examples, infrared spectra of the molecular sieves were acquired by using an iS50 infrared spectrometer (available from Thermo Scientific, USA), potassium bromide tablets were used, and the tested wavenumber range was 400-4000 cm⁻¹.

### Example 1

This example is used to illustrate the synthesis of a ZSM-5 molecular sieve and the preparation of a catalyst for the alkylation of benzene to ethylbenzene.
(1) 208.3 g of tetraethyl orthosilicate, 3.33 g of aluminum sulfate 18 hydrate, 180 g of water, 101.68 g of tetrapropylammonium hydroxide and 19.13 g of organic matter II of formula 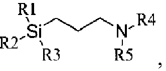 where R1 is methoxy, both R2 and R3 are ethoxy, and both R4 and R5 are hydrogen, were uniformly mixed to obtain a mixture A1. Thus, a molar ratio of the tetraethyl orthosilicate in terms of SiO₂ to the aluminum sulfate 18 hydrate in terms of Al₂O₃ to the water to the tetrapropylammonium hydroxide to the organic matter II was 1 : 0.005 : 10 : 0.5 : 0.1.
(2) The mixture A1 was stirred in a closed container at 75 °C for 18 h to obtain a mixture B1.
(3) 26.6 g of the mixture B1, 60 g of silicon powder, 3.33 g of aluminum sulfate 18 hydrate, 180 g of water, 9.02 g of ethylamine and 14.25 g of organic matter III of formula 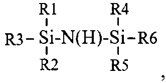 where both R1 and R4 are phenyl, and R2, R3, R5 and R6 each are methyl, were brought into contact to obtain a mixture C1. Thus, a molar ratio of the silicon powder in terms of SiO₂ to the aluminum sulfate 18 hydrate in terms of Al₂O₃ to the water to the ethylamine to the organic matter III was 1 : 0.005 : 10 : 0.2 : 0.05.
(4) The mixture C1 was allowed to crystallize in a closed container at 150° C for 48 h to obtain a mixture D1. The mixture D1 was then centrifuged, and the obtained solids were washed with deionized water several times until the washing solution reached pH 7.5. Then, the washed solids were dried in an oven at 150 °C for 10 h to obtain a molecular sieve E1.
(5) 50 g of the E1, 15 g of aluminum oxide monohydrate, 30 g of 5 mass% nitric acid aqueous solution, 1.5 g of sesbania powder and 5 g of a polymer mixture (composed of 3 g of starch and 2 g of hexadecyltrimethylammonium bromide) were mixed and then extruded into a cylindrical shape. Next, the extrudes were treated at 150 °C for 8 hours and then at 550 °C for 6 hours; subjected 4 times to an exchanging treatment with a 5 mass% ammonium sulfate solution at 50 °C for 6 hours; treated under saturated water vapor atmosphere at 400 °C for 6 hours; and then treated with 0.5 mass% hydrochloric acid solution at 40 °C for 5 hours, to obtain a catalyst F1.

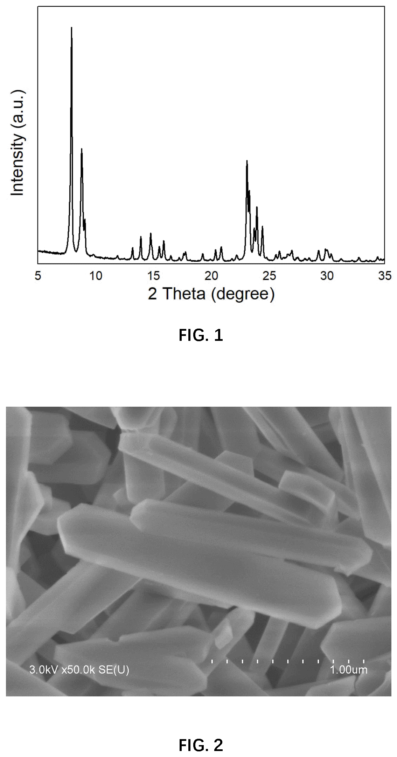

An XRD pattern of the molecular sieve E1 is shown in Figure 1, which has typical diffraction peaks of a ZSM-5 molecular sieve.

An SEM photograph of the molecular sieve E1 is shown in Figure 2. There were no twin crystals in the molecular sieve. According to statistics, the size of the molecular sieve in the a-axis direction was La = 293 nm, the size in the b-axis direction was Lb = 84 nm, and the size in the c-axis direction was Lc = 1970 nm; the size of the crystal edge formed by the intersection of (010) and (101) crystal faces was Ld = 190 nm, and the size of the crystal edge formed by the intersection of (010) and (100) crystal faces was Le = 1728 nm; a ratio of the size in the a-axis direction to the size in the b-axis direction was La/Lb = 3.5, and a ratio of the size in the c-axis direction to the size in the b-axis direction was Lc/Lb = 23.5.

For the molecular sieve, the area of the (100) crystal face was S1 = 145152 nm², the area of the (010) crystal face was S2 = 541757 nm², and the area of the (101) crystal face was S3 = 15960 nm²; a ratio of the area of the (100) crystal face to the area of the (010) crystal face was S1/S2 = 0.27, a ratio of the area of the (101) crystal face to the area of the (010) crystal face was S3/S2 = 0.029; and the exposure degree of the (010) crystal face was S2/(S1+S2+2S3)×100% = 75.4%.

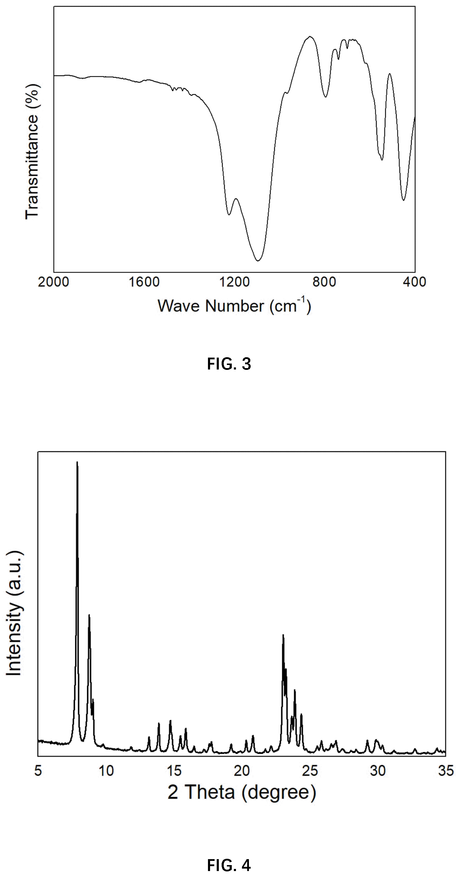

The infrared spectrum of the molecular sieve E1 is shown in Figure 4. Two peaks appeared in a wavenumber range of 690 to 750 cm⁻¹, with the wavenumbers corresponding to the peak vertices being 700 cm⁻¹ and 739 cm⁻¹, respectively. Two peaks appeared in a wavenumber range of 440 to 560 cm⁻¹, with the wavenumbers corresponding to the peak vertices being 450 cm⁻¹ and 550 cm⁻¹, respectively. The molecular sieve E1 had a SiO₂/Al₂O₃ molar ratio of 200.

### Example 2

152.2 g of tetramethyl orthosilicate, 2.66 g of aluminum sulfate 18 hydrate, 360 g of water, 73.1 g of n-butylamine and 70.8 g of organic matter II of formula 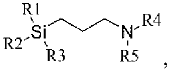 where both R1 and R2 are methoxy, R3 is ethoxy, R4 is 2-aminoethyl, and R5 is hydrogen, were uniformly mixed to obtain a mixture A2. Thus, a molar ratio of the tetramethyl orthosilicate in terms of SiO₂ to the aluminum sulfate 18 hydrate in terms of Al₂O₃ to the water to the n-butylamine to the organic matter II was 1 : 0.004 : 20 : 1 : 0.3. The mixture A2 was stirred in a closed container at 85 °C for 8 h to obtain a mixture B2. 101 g of the mixture B2, 60 g of white carbon black, 0.408 g of pseudo-boehmite, 360 g of water, 61 g of tetrapropylammonium hydroxide and 38.9 g of organic matter III of formula 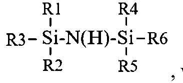 where R1-R3 each are phenyl, and R4-R6 each are ethyl, were brought into contact to obtain a mixture C2. Thus, a molar ratio of the white carbon black in terms of SiO₂ to the pseudo-boehmite in terms of Al₂O₃ to the water to the tetrapropylammonium hydroxide to the organic matter III was 1 : 0.004 : 20 : 0.3 : 0.1. The mixture C2 was allowed to crystallize in a closed container at 170 °C for 12 h to obtain a mixture D2. The mixture D2 was then centrifuged, and the obtained solids were washed with deionized water several times until the washing solution reached pH 7.5. Then, the washed solids were dried in an oven at 200 °C for 5 h to obtain a molecular sieve E2.

50 g of the E2, 20 g of aluminum oxide monohydrate, 60 g of 3 mass% nitric acid aqueous solution, 2.5 g of sesbania powder and 10 g of a polymer mixture (composed of 7 g of starch and 3 g of P123) were mixed and then extruded into a four-lobed shape. Next, the extrudes were treated at 200 °C for 5 hours and then at 600 °C for 4 hours; subjected 4 times to an exchanging treatment with a 10 mass% ammonium chloride solution at 80 °C for 1 hour; treated under saturated water vapor atmosphere at 450 °C for 4 hours; and then treated with 3 mass% hydrochloric acid solution at 80 °C for 1 hour, to obtain a catalyst F2.

The XRD characterization of the molecular sieve E2 showed that it had typical diffraction peaks of a ZSM-5 molecular sieve.

SEM photograph showed that there were no twin crystals in the molecular sieve E2. According to statistics, the size of the molecular sieve in the a-axis direction was La = 270 nm, the size in the b-axis direction was Lb = 60 nm, and the size in the c-axis direction was Lc = 1560 nm; the size of the crystal edge formed by the intersection of (010) and (101) crystal faces was Ld = 180 nm, and the size of the crystal edge formed by the intersection of (010) and (100) crystal faces was Le = 1322 nm; a ratio of the size in the a-axis direction to the size in the b-axis direction was La/Lb = 4.5, and a ratio of the size in the c-axis direction to the size in the b-axis direction was Lc/Lb = 26.0.

For the molecular sieve, the area of the (100) crystal face was S1 = 79320 nm², the area of the (010) crystal face was S2 = 389070 nm², and the area of the (101) crystal face was S3 = 10800 nm²; a ratio of the area of the (100) crystal face to the area of the (010) crystal face was S1/S2 = 0.20, a ratio of the area of the (101) crystal face to the area of the (010) crystal face was S3/S2 = 0.028; and the exposure degree of the (010) crystal face was S2/(S1+S2+2S3)×100% = 79.4%.

In an infrared spectrum of the molecular sieve E2, two peaks appeared in a wavenumber range of 690 to 750 cm⁻¹, with the wavenumbers corresponding to the peak vertices being 700 cm⁻¹ and 740 cm⁻¹, respectively, and two peaks appeared in a wavenumber range of 440 to 560 cm⁻¹, with the wavenumbers corresponding to the peak vertices being 450 cm⁻¹ and 550 cm⁻¹, respectively. The molecular sieve E2 had a SiO₂/Al₂O₃ molar ratio of 250.

### Example 3

208.3 g of tetraethyl orthosilicate, 2.20 g of aluminum sulfate 18 hydrate, 90 g of water, 20.34 g of tetrapropylammonium hydroxide and 16.05 g of organic matter II of formula 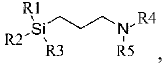 where R1 is methoxy, R2 is ethoxy, R3 is 3-methoxypropyl, and both R4 and R5 are methyl, were uniformly mixed to obtain a mixture A3. Thus, a molar ratio of the tetraethyl orthosilicate in terms of SiO₂ to the aluminum sulfate 18 hydrate in terms of Al₂O₃ to the water to the tetrapropylammonium hydroxide to the organic matter II was 1 : 0.0033 : 5 : 0.1 : 0.05. The mixture A3 was stirred in a closed container at 55 °C for 24 h to obtain a mixture B3. 18.23 g of the mixture B3, 208.3 g of tetraethyl orthosilicate, 2.20 g of aluminum sulfate 18 hydrate, 144 g of water, 7.34 g of n-butylamine and 4.09 g of organic matter III of formula 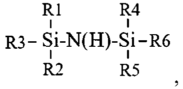 where R1, R2, R4 and R5 are phenyl, and R3 and R6 are methyl, were brought into contact to obtain a mixture C3. Thus, a molar ratio of the tetraethyl orthosilicate in terms of SiO₂ to the aluminum sulfate 18 hydrate in terms of Al₂O₃ to the water to the n-butylamine to the organic matter III was 1 : 0.0033 : 8 : 0.1 : 0.01. The mixture C3 was allowed to crystallize in a closed container at 110° C for 72 h to obtain a mixture D3. The mixture D3 was then centrifuged, and the obtained solids were washed with deionized water several times until the washing solution reached pH 7.5. Then, the washed solids were dried in an oven at 100 °C for 12 h to obtain a molecular sieve E3.

50 g of the E3, 5 g of aluminum oxide monohydrate, 25 g of 3 mass% nitric acid aqueous solution, 0.5 g of sesbania powder and 1.5 g of a polymer mixture (composed of 0.825 g of starch and 0.675 g of F127) were mixed and then extruded into a four-lobed shape. Next, the extrudes were treated at 100 °C for 12 hours and then at 500 °C for 10 hours; subjected 4 times to an exchanging treatment with a 2 mass% ammonium acetate solution at 20 °C for 10 hours; treated under saturated water vapor atmosphere at 300 °C for 10 hours; and then treated with 0.1 mass% oxalic acid solution at 20 °C for 10 hours, to obtain a catalyst F3.

The XRD characterization of the molecular sieve E3 showed that it had typical diffraction peaks of a ZSM-5 molecular sieve.

SEM photograph showed that there were no twin crystals in the molecular sieve E3. According to statistics, the size of the molecular sieve in the a-axis direction was La = 240 nm, the size in the b-axis direction was Lb = 90 nm, and the size in the c-axis direction was Lc = 2200 nm; the size of the crystal edge formed by the intersection of (010) and (101) crystal faces was Ld = 220 nm, and the size of the crystal edge formed by the intersection of (010) and (100) crystal faces was Le = 1831 nm; a ratio of the size in the a-axis direction to the size in the b-axis direction was La/Lb = 2.7, and a ratio of the size in the c-axis direction to the size in the b-axis direction was Lc/Lb = 24.4.

For the molecular sieve, the area of the (100) crystal face was S1 = 164790 nm², the area of the (010) crystal face was S2 = 483720 nm², and the area of the (101) crystal face was S3 = 19800 nm²; a ratio of the area of the (100) crystal face to the area of the (010) crystal face was S1/S2 = 0.34, a ratio of the area of the (101) crystal face to the area of the (010) crystal face was S3/S2 = 0.041; and the exposure degree of the (010) crystal face was S2/(S1+S2+2S3)×100% = 70.3%.

In an infrared spectrum of the molecular sieve E3, two peaks appeared in a wavenumber range of 690 to 750 cm⁻¹, with the wavenumbers corresponding to the peak vertices being 700 cm⁻¹ and 739 cm⁻¹, respectively, and two peaks appeared in a wavenumber range of 440 to 560 cm⁻¹, with the wavenumbers corresponding to the peak vertices being 449 cm⁻¹ and 550 cm⁻¹, respectively. The molecular sieve E3 had a SiO₂/Al₂O₃ molar ratio of 298.

### Example 4

208.3 g of tetraethyl orthosilicate, 180 g of water, 101.68 g of tetrapropylammonium hydroxide and 19.13 g of organic matter II of formula 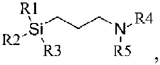 where R1 is methoxy, both R2 and R3 are ethoxy, and both R4 and R5 are hydrogen, were uniformly mixed to obtain a mixture A4. Thus, a molar ratio of the tetraethyl orthosilicate in terms of SiO₂ to the water to the tetrapropylammonium hydroxide to the organic matter II was 1 : 10 : 0.5 : 0.1. The mixture A4 was stirred in a closed container at 80 °C for 10 h to obtain a mixture B4. 26.6 g of the mixture B4, 60 g of silicon powder, 3.33 g of aluminum sulfate 18 hydrate, 180 g of water, 9.02 g of ethylamine and 14.25 g of organic matter III of formula 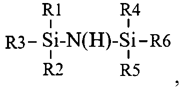 where R1 and R4 are phenyl, and R2, R3, R5 and R6 are methyl, were brought into contact to obtain a mixture C4. Thus, a molar ratio of the silicon powder in terms of SiO₂ to the aluminum sulfate 18 hydrate in terms of Al₂O₃ to the water to the ethylamine to the organic matter III was 1 : 0.005 : 10 : 0.2 : 0.05. The mixture C4 was allowed to crystallize in a closed container at 150° C for 48 h to obtain a mixture D4. The mixture D4 was then centrifuged, and the obtained solids were washed with deionized water several times until the washing solution reached pH 7.5. Then, the washed solids were dried in an oven at 150 °C for 10 h to obtain a molecular sieve E4.

50 g of the E4, 15 g of aluminum oxide monohydrate, 30 g of 5 mass% nitric acid aqueous solution, 1.5 g of sesbania powder and 5 g of a polymer mixture (composed of 3 g of starch and 2 g of hexadecyltrimethylammonium bromide) were mixed and then extruded into a cylindrical shape. Next, the extrudes were treated at 150 °C for 8 hours and then at 550 °C for 6 hours; subjected 4 times to an exchanging treatment with a 5 mass% ammonium sulfate solution at 50 °C for 6 hours; treated under saturated water vapor atmosphere at 400 °C for 6 hours; and then treated with 0.5 mass% hydrochloric acid solution at 40 °C for 5 hours, to obtain a catalyst F4.

The XRD characterization of the molecular sieve E4 showed that it had typical diffraction peaks of a ZSM-5 molecular sieve.

SEM photograph showed that there were no twin crystals in the molecular sieve E4. According to statistics, the size of the molecular sieve in the a-axis direction was La = 245 nm, the size in the b-axis direction was Lb = 80 nm, and the size in the c-axis direction was Lc = 2000 nm; the size of the crystal edge formed by the intersection of (010) and (101) crystal faces was Ld = 215 nm, and the size of the crystal edge formed by the intersection of (010) and (100) crystal faces was Le = 1646 nm; a ratio of the size in the a-axis direction to the size in the b-axis direction was La/Lb = 3.1, and a ratio of the size in the c-axis direction to the size in the b-axis direction was Lc/Lb = 25.0.

For the molecular sieve, the area of the (100) crystal face was S1 = 131680 nm², the area of the (010) crystal face was S2 = 446635 nm², and the area of the (101) crystal face was S3 = 17200 nm²; a ratio of the area of the (100) crystal face to the area of the (010) crystal face was S1/S2 = 0.29, a ratio of the area of the (101) crystal face to the area of the (010) crystal face was S3/S2 = 0.039; and the exposure degree of the (010) crystal face was S2/(S1+S2+2S3)×100% = 72.9%.

In an infrared spectrum of the molecular sieve E4, two peaks appeared in a wavenumber range of 690 to 750 cm⁻¹, with the wavenumbers corresponding to the peak vertices being 700 cm⁻¹ and 740 cm⁻¹, respectively, and two peaks appeared in a wavenumber range of 440 to 560 cm⁻¹, with the wavenumbers corresponding to the peak vertices being 450 cm⁻¹ and 549 cm⁻¹, respectively. The molecular sieve E4 had a SiO₂/Al₂O₃ molar ratio of 202.

### Comparative Example 1

A molecular sieve E5 and further a catalyst F5 were prepared by following the procedure described in Example 1, except that the organic matter II of formula 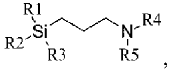 where R1 is methoxy, both R2 and R3 are ethoxy, and both R4 and R5 are hydrogen, was not added.

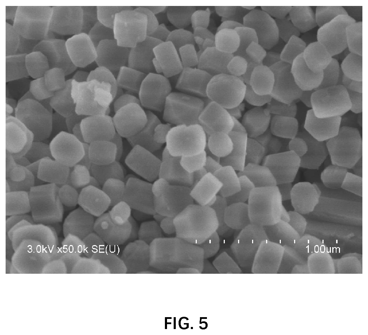

An XRD pattern of the molecular sieve E5 is shown in figure 5, which has typical diffraction peaks of a ZSM-5 molecular sieve.

An SEM photograph of the molecular sieve E5 is shown in Figure 6. There were no twin crystals in the molecular sieve. According to statistics, the size of the molecular sieve in the a-axis direction was La = 238 nm, the size in the b-axis direction was Lb = 160 nm, and the size in the c-axis direction was Lc = 286 nm; the size of the crystal edge formed by the intersection of (010) and (101) crystal faces was Ld = 152 nm, and the size of the crystal edge formed by the intersection of (010) and (100) crystal faces was Le = 97 nm; a ratio of the size in the a-axis direction to the size in the b-axis direction was La/Lb = 1.5, and a ratio of the size in the c-axis direction to the size in the b-axis direction was Lc/Lb = 1.8.

For the molecular sieve, the area of the (100) crystal face was S1 = 15520 nm², the area of the (010) crystal face was S2 = 45577 nm², and the area of the (101) crystal face was S3 = 24320 nm²; a ratio of the area of the (100) crystal face to the area of the (010) crystal face was S1/S2 = 0.34, a ratio of the area of the (101) crystal face to the area of the (010) crystal face was S3/S2 = 0.534; and the exposure degree of the (010) crystal face was S2/(S1+S2+2S3)×100% = 41.5%.

In an infrared spectrum of the molecular sieve E5, two peaks appeared in a wavenumber range of 690 to 750 cm⁻¹, with the wavenumbers corresponding to the peak vertices being 700 cm⁻¹ and 740 cm⁻¹, respectively, and two peaks appeared in a wavenumber range of 440 to 560 cm⁻¹, with the wavenumbers corresponding to the peak vertices being 450 cm⁻¹ and 550 cm⁻¹, respectively. The molecular sieve E5 had a SiO₂/Al₂O₃ molar ratio of 202.

### Comparative Example 2

A molecular sieve E6 and further a catalyst F6 were prepared by following the procedure described in Example 1, except that the organic matter III of formula 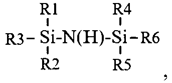 where both R1 and R4 are phenyl, and R2, R3, R5 and R6 each are methyl, was not added.

The XRD characterization of the molecular sieve E6 showed that it had typical diffraction peaks of a ZSM-5 molecular sieve.

An SEM photograph showed that there were no twin crystals in the molecular sieve E6. According to statistics, the size of the molecular sieve in the a-axis direction was La = 360 nm, the size in the b-axis direction was Lb = 185 nm, and the size in the c-axis direction was Lc = 2100 nm; the size of the crystal edge formed by the intersection of (010) and (101) crystal faces was Ld = 228 nm, and the size of the crystal edge formed by the intersection of (010) and (100) crystal faces was Le = 1820 nm; a ratio of the size in the a-axis direction to the size in the b-axis direction was La/Lb = 1.9, and a ratio of the size in the c-axis direction to the size in the b-axis direction was Lc/Lb = 11.4.

For the molecular sieve, the area of the (100) crystal face was S1 = 336700 nm², the area of the (010) crystal face was S2 = 705600 nm², and the area of the (101) crystal face was S3 = 42180 nm²; a ratio of the area of the (100) crystal face to the area of the (010) crystal face was S1/S2 = 0.48, a ratio of the area of the (101) crystal face to the area of the (010) crystal face was S3/S2 = 0.060; and the exposure degree of the (010) crystal face was S2/(S1+S2+2S3)×100% = 62.6%.

In an infrared spectrum of the molecular sieve E6, there were not two peaks appearing in a wavenumber range of 690 to 750 cm⁻¹. The molecular sieve E6 had a SiO₂/Al₂O₃ molar ratio of 201.

### Comparative Example 3

A molecular sieve E7 and further a catalyst F7 were prepared by following the procedure described in Example 1, except that 0.995 g of the organic matter II and 14.25 g of the organic matter III were added simultaneously in step (3).

The XRD characterization of the molecular sieve E7 showed that it had typical diffraction peaks of a ZSM-5 molecular sieve.

An SEM photograph showed that there were no twin crystals in the molecular sieve E7. According to statistics, the size of the molecular sieve in the a-axis direction was La = 234 nm, the size in the b-axis direction was Lb = 155 nm, and the size in the c-axis direction was Lc = 368 nm; the size of the crystal edge formed by the intersection of (010) and (101) crystal faces was Ld = 185 nm, and the size of the crystal edge formed by the intersection of (010) and (100) crystal faces was Le = 82 nm; a ratio of the size in the a-axis direction to the size in the b-axis direction was La/Lb = 1.5, and a ratio of the size in the c-axis direction to the size in the b-axis direction was Lc/Lb = 2.4.

For the molecular sieve, the area of the (100) crystal face was S1 = 12710 nm², the area of the (010) crystal face was S2 = 52650 nm², and the area of the (101) crystal face was S3 = 28675 nm²; a ratio of the area of the (100) crystal face to the area of the (010) crystal face was S1/S2 = 0.24, a ratio of the area of the (101) crystal face to the area of the (010) crystal face was S3/S2 = 0.545; and the exposure degree of the (010) crystal face was S2/(S1+S2+2S3)×100% = 42.9%.

In an infrared spectrum of the molecular sieve E7, two peaks appeared in a wavenumber range of 690 to 750 cm⁻¹, with the wavenumbers corresponding to the peak vertices being 700 cm⁻¹ and 740 cm⁻¹, respectively, and two peaks appeared in a wavenumber range of 440 to 560 cm⁻¹, with the wavenumbers corresponding to the peak vertices being 449 cm⁻¹ and 549 cm⁻¹, respectively. The molecular sieve E7 had a SiO₂/Al₂O₃ molar ratio of 202.

### Comparative Example 4

A molecular sieve E8 and further a catalyst F8 were prepared by following the procedure described in Example 1, except that in step (3), the organic matter III of formula 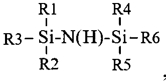 where both R1 and R4 are phenyl, and R2, R3, R5 and R6 each are methyl, was replaced with an equal molar amount (9.57 g) of the organic matter II of formula 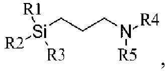 where R1 is methoxy, both R2 and R3 are ethoxy, and both R4 and R5 are hydrogen.

The XRD characterization of the molecular sieve E8 showed that it had typical diffraction peaks of a ZSM-5 molecular sieve.

An SEM photograph showed that there were no twin crystals in the molecular sieve E8. According to statistics, the size of the molecular sieve in the a-axis direction was La = 325 nm, the size in the b-axis direction was Lb = 130 nm, and the size in the c-axis direction was Lc = 2052 nm; the size of the crystal edge formed by the intersection of (010) and (101) crystal faces was Ld = 255 nm, and the size of the crystal edge formed by the intersection of (010) and (100) crystal faces was Le = 1658 nm; a ratio of the size in the a-axis direction to the size in the b-axis direction was La/Lb = 2.5, and a ratio of the size in the c-axis direction to the size in the b-axis direction was Lc/Lb = 15.8.

For the molecular sieve, the area of the (100) crystal face was S1 = 215540 nm², the area of the (010) crystal face was S2 = 602875 nm², and the area of the (101) crystal face was S3 = 33150 nm²; a ratio of the area of the (100) crystal face to the area of the (010) crystal face was S1/S2 = 0.36, a ratio of the area of the (101) crystal face to the area of the (010) crystal face was S3/S2 = 0.055; and the exposure degree of the (010) crystal face was S2/(S1+S2+2S3)×100% = 68.1%.

In an infrared spectrum of the molecular sieve E8, there were not two peaks appearing in a wavenumber range of 690 to 750 cm⁻¹. The molecular sieve E8 had a SiO₂/Al₂O₃ molar ratio of 201.

### Comparative Example 5

A molecular sieve E9 and further a catalyst F9 were prepared by following the procedure described in Example 1, except that in step (1), the organic matter II of formula 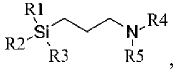 where R1 is methoxy, both R2 and R3 are ethoxy, and both R4 and R5 are hydrogen, was replaced with an equal molar amount (28.5 g) of the organic matter III of formula 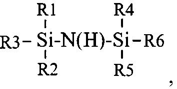 where both R1 and R4 are phenyl, and R2, R3, R5 and R6 each are methyl.

The XRD characterization of the molecular sieve E9 showed that it had typical diffraction peaks of a ZSM-5 molecular sieve.

An SEM photograph showed that there were no twin crystals in the molecular sieve E9. According to statistics, the size of the molecular sieve in the a-axis direction was La = 216 nm, the size in the b-axis direction was Lb = 148 nm, and the size in the c-axis direction was Lc = 827 nm; the size of the crystal edge formed by the intersection of (010) and (101) crystal faces was Ld = 170 nm, and the size of the crystal edge formed by the intersection of (010) and (100) crystal faces was Le = 564 nm; a ratio of the size in the a-axis direction to the size in the b-axis direction was La/Lb = 1.5, and a ratio of the size in the c-axis direction to the size in the b-axis direction was Lc/Lb = 5.6.

For the molecular sieve, the area of the (100) crystal face was S1 = 83472 nm², the area of the (010) crystal face was S2 = 150228 nm², and the area of the (101) crystal face was S3 = 25160 nm²; a ratio of the area of the (100) crystal face to the area of the (010) crystal face was S1/S2 = 0.56, a ratio of the area of the (101) crystal face to the area of the (010) crystal face was S3/S2 = 0.167; and the exposure degree of the (010) crystal face was S2/(S1+S2+2S3)×100% = 52.9%.

In an infrared spectrum of the molecular sieve E9, two peaks appeared in a wavenumber range of 690 to 750 cm⁻¹, with the wavenumbers corresponding to the peak vertices being 700 cm⁻¹ and 740 cm⁻¹, respectively, and two peaks appeared in a wavenumber range of 440 to 560 cm⁻¹, with the wavenumbers corresponding to the peak vertices being 449 cm⁻¹ and 549 cm⁻¹, respectively. The molecular sieve E9 had a SiO₂/Al₂O₃ molar ratio of 201.

### Comparative Example 6

A molecular sieve E10 and further a catalyst F10 were prepared by following the procedure described in Example 1, except that in step (2), the mixture A was stirred in a closed container at 90 °C for 18 h.

The XRD characterization of the molecular sieve E10 showed that it had typical diffraction peaks of a ZSM-5 molecular sieve.

An SEM photograph showed that there were no twin crystals in the molecular sieve E10. According to statistics, the size of the molecular sieve in the a-axis direction was La = 305 nm, the size in the b-axis direction was Lb = 92 nm, and the size in the c-axis direction was Lc = 1450 nm; the size of the crystal edge formed by the intersection of (010) and (101) crystal faces was Ld = 261 nm, and the size of the crystal edge formed by the intersection of (010) and (100) crystal faces was Le = 1026 nm; a ratio of the size in the a-axis direction to the size in the b-axis direction was La/Lb = 3.3, and a ratio of the size in the c-axis direction to the size in the b-axis direction was Lc/Lb = 15.8.

For the molecular sieve, the area of the (100) crystal face was S1 = 94392 nm², the area of the (010) crystal face was S2 = 377590 nm², and the area of the (101) crystal face was S3 = 24012 nm²; a ratio of the area of the (100) crystal face to the area of the (010) crystal face was S1/S2 = 0.25, a ratio of the area of the (101) crystal face to the area of the (010) crystal face was S3/S2 = 0.064; and the exposure degree of the (010) crystal face was S2/(S1+S2+2S3)×100% = 72.6%.

In an infrared spectrum of the molecular sieve E10, two peaks appeared in a wavenumber range of 690 to 750 cm⁻¹, with the wavenumbers corresponding to the peak vertices being 700 cm⁻¹ and 739 cm⁻¹, respectively, and two peaks appeared in a wavenumber range of 440 to 560 cm⁻¹, with the wavenumbers corresponding to the peak vertices being 450 cm⁻¹ and 550 cm⁻¹, respectively. The molecular sieve E10 had a SiO₂/Al₂O₃ molar ratio of 201.

### Examples 5-8

The catalysts F1-F4 prepared in Examples 1-4 were respectively used in the alkylation of benzene to produce ethylbenzene. Under conditions of an ethylene mass concentration of 50%, a reaction temperature of 340 °C, a pressure of 2.2 MPa, an ethylene mass space velocity of 0.8 h⁻¹ and a molar ratio of benzene to ethylene of 4.0, ethylene conversion rate, selectivity to ethyl product in the alkylation product, and content of xylenes as key impurities were tested. The results are shown in Table 1 below.

### Comparative Examples 6-10

The catalysts F5-F10 prepared in Comparative Examples 1-6 were respectively used in the alkylation of benzene to produce ethylbenzene. Under conditions of an ethylene mass concentration of 50%, a reaction temperature of 340 °C, a pressure of 2.2 MPa, an ethylene mass space velocity of 0.8 h⁻¹ and a molar ratio of benzene to ethylene of 4.0, ethylene conversion rate, selectivity to ethyl product in the alkylation product, and content of xylenes as key impurities were tested. The results are shown in Table 1 below.

**Table 1. Reaction results of the alkylation of benzene to produce ethylbenzene**

| Catalyst No. | Ethylene Conversion Rate, wt.% | Selectivity to Ethyl Product, wt.% | Content of Xylenes, ppm by mass |
|---|---|---|---|
| F1 | 100.0 | 99.8 | 452 |
| F2 | 100.0 | 99.8 | 408 |
| F3 | 100.0 | 99.7 | 579 |
| F4 | 100.0 | 99.8 | 480 |
| F5 | 99.1 | 98.7 | 1958 |
| F6 | 99.3 | 99.0 | 1499 |
| F7 | 99.6 | 99.2 | 1276 |
| F8 | 99.6 | 99.3 | 925 |
| F9 | 99.6 | 99.2 | 1180 |
| F10 | 99.7 | 99.5 | 786 |

### Examples 9-12

The catalysts F1-F4 prepared in Examples 1-4 were tested for their deactivation time in alkylation of benzene to produce ethylbenzene under strict reaction conditions including an ethylene mass concentration of 50%, a reaction temperature of 400 °C, a pressure of 2.2 MPa, an ethylene mass space velocity of 20 h⁻¹ and a molar ratio of benzene to ethylene of 2.0. The deactivation time refers to a period of time from the start of the reaction to the time when the ethylene conversion rate drops to 40% of the initial conversion rate. The results are shown in Table 2 below.

### Comparative Examples 11-15

The catalysts F5-F10 prepared in Comparative Examples 1-5 were tested for their deactivation time in alkylation of benzene to produce ethylbenzene under strict reaction conditions including an ethylene mass concentration of 50%, a reaction temperature of 400 °C, a pressure of 2.2 MPa, an ethylene mass space velocity of 20 h⁻¹ and a molar ratio of benzene to ethylene of 2.0. The deactivation time refers to a period of time from the start of the reaction to the time when the ethylene conversion rate drops to 40% of the initial conversion rate. The results are shown in Table 2 below.

**Table 2. Test results of catalyst deactivation time under ultra-high space velocity reaction conditions**

| Catalyst No. | Deactivation Time, h |
|---|---|
| F1 | 368 |
| F2 | 382 |
| F3 | 346 |
| F4 | 355 |
| F5 | 173 |
| F6 | 198 |
| F7 | 206 |
| F8 | 243 |
| F9 | 238 |
| F10 | 301 |

Under the ultra-high ethylene space velocity reaction conditions, the deactivation time of the catalysts F1-F4 is significantly longer than that of the catalysts F5-F10.

Specific implementations of the present invention have been described in detail above, but the present invention is not limited thereto. Within the technical concept of the present invention, the technical solutions of the present invention can be subjected to a variety of simple modifications, including combinations of the various technical features in any other suitable manner. Such simple modifications and combinations should also be regarded as the contents disclosed by the present invention and belong to the protection scope of the present invention.

## Claims

1. AZSM-5 molecular sieve, **characterized in that** there are no twin crystals in the molecular sieve, and the molecular sieve has a size in a-axis direction, La, of 240 to 300 nm, a size in b-axis direction, Lb, of 60 to 90 nm, and a size in c-axis direction, Lc, of 1500 to 2200 nm,
for the molecular sieve, a size of a crystal edge formed by the intersection of (010) and (101) crystal faces, Ld, is from 180 to 220 nm, and a size of a crystal edge formed by the intersection of (010) and (100) crystal faces, Le, is from 1300 to 1900 nm, and
for the molecular sieve, a ratio of the a-axis direction size to the b-axis direction size, La/Lb, is from 2.5 to 4.5, and a ratio of the c-axis direction size to the b-axis direction size, Lc/Lb, is from 22.0 to 26.0.

2. The molecular sieve according to claim 1, **characterized in that** for the molecular sieve, an area of the (100) crystal face, S1, is from 75,000 to 180,000 nm², an area of the (010) crystal face, S2, is from 350,000 to 600,000 nm², and an area of the (101) crystal face, S3, is from 10,000 to 20,000 nm²,
for the molecular sieve, a ratio of the (100) crystal face area to the (010) crystal face area, S1/S2, is from 0.20 to 0.35, and a ratio of the (101) crystal face area to the (010) crystal face area, S3/S2, is from 0.025 to 0.045, and
for the molecular sieve, an exposure degree of the (010) crystal face, defined as S2/(S1+S2+2S3)×100%, is from 70 to 80 %.

3. The molecular sieve according to claim 1 or 2, **characterized in that** an infrared spectrum of the molecular sieve has two peaks P1 and P2 in a wavenumber range of 690-750 cm⁻¹, with the vertexes of the peaks P1 and P2 falling within wavenumber ranges of 690-710 cm⁻¹ and 730-750 cm⁻¹, respectively,
the infrared spectrum of the molecular sieve has two peaks P3 and P4 in a wavenumber range of 440-560 cm⁻¹, with the vertexes of the peaks P3 and P4 falling within wavenumber ranges of 440-460 cm⁻¹ and 540-560 cm⁻¹, respectively, and
the molecular sieve has a SiO₂:Al₂O₃ molar ratio of 100-300.

4. The molecular sieve according to claim 1, **characterized in that** the molecular sieve in an as-synthesized state has a chemical composition, in moles, including: SiO₂:nAl₂O₃:mE2:xE3, where E2 represents an organic matter II, E3 represents an organic matter III, n is a value of 0.0033 to 0.01, m is a value of 0.0015 to 0.005, and x is a value of 0.005 to 0.1,
the molecular sieve in the as-synthesized state optionally comprises a template and water, wherein a molar ratio of the template to SiO₂ is from 0.05 to 0.3, and a molar ratio of the water to SiO₂ is from 3 to 20,
wherein the organic matter II has a structural formula 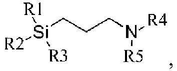 wherein R1-R3 are each independently selected from the group consisting of C1-C6 alkoxy and C1-C6 alkoxy-C1-C6 alkyl, and preferably methoxy, ethoxy or 3-methoxypropyl, and R4 and R5 are each independently selected from the group consisting of hydrogen, C1-C6 alkyl and amino-C1-C6 alkyl, and preferably hydrogen, methyl, ethyl, aminomethyl or aminoethyl, and
the organic matter III has a structural formula 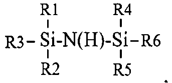 wherein R1-R6 are each independently selected from the group consisting of methyl, ethyl, propyl and phenyl, provided that one, two, three or four of R1-R6 are phenyl.

5. A method for preparing the ZSM-5 molecular sieve according to any one of claims 1 to 4, comprising:
(a) mixing a silicon source I, an optional aluminum source I, water, an organic matter I and an organic matter II to obtain a mixture A;
(b) treating the mixture A to obtain a mixture B having a morphology-directing effect;
(c) mixing the mixture B, a silicon source II, an aluminum source II, water, a template and an organic matter III to obtain a mixture C; and
(d) allowing the mixture C to crystallize, followed by liquid-solid separation, washing and drying, to obtain the molecular sieve,
wherein the organic matter I is one or more of tetrapropylammonium hydroxide, ethylamine, ethylenediamine, n-butylamine, tetramethylendiamine, hexamethyleneimine, and piperidine;
wherein the organic matter II has a structural formula 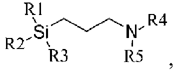 wherein R1-R3 are each independently selected from the group consisting of C1-C6 alkoxy and C1-C6 alkoxy-C1-C6 alkyl, and preferably methoxy, ethoxy or 3-methoxypropyl, and R4 and R5 are each independently selected from the group consisting of hydrogen, C1-C6 alkyl and amino-C1-C6 alkyl, and preferably hydrogen, methyl, ethyl, aminomethyl or aminoethyl; and
wherein the organic matter III has a structural formula 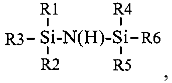
wherein R1-R6 are each independently selected from the group consisting of methyl, ethyl, propyl and phenyl, provided that one, two, three or four of R1-R6 are phenyl.

6. The method according to claim 4, **characterized in that** the silicon source I in step (a) is one or more of tetramethyl orthosilicate, tetraethyl orthosilicate, tetrapropyl orthosilicate, tetrabutyl orthosilicate and tetraphenyl orthosilicate; and/or the aluminum source I in step (a) is one or more of aluminum sulfate, aluminum isopropoxide, sodium aluminate, and pseudo-boehmite;
preferably, in step (a), a molar ratio of the silicon source I in terms of SiO₂ to the aluminum source I in terms of Al₂O₃ to the water to the organic matter I to the organic matter II is 1 : (0-0.02) : (5-20) : (0.1-1.0) : (0.05-0.3).

7. The method according to claim 4, **characterized in that** the treating in step (b) is carried out at 55-85 °C under closed conditions with stirring for 6-24 hours; and/or in step (d), the crystallizing is carried out by crystallizing under closed conditions at 110-170 °C for 12-72 hours.

8. The method according to claim 4, **characterized in that** the silicon source II in step (c) is one or more of silica sol, white carbon black, silicon powder, tetramethyl orthosilicate, tetraethyl orthosilicate, tetrapropyl orthosilicate, tetrabutyl orthosilicate and tetraphenyl orthosilicate; and/or the aluminum source II in step (c) is one or more of aluminum nitrate, aluminum chloride, aluminum sulfate, aluminum isopropoxide, sodium aluminate, and pseudo-boehmite; and/or the template in step (c) is one or more of tetrapropylammonium bromide, tetrapropylammonium hydroxide, ethylamine, ethylenediamine, n-propylamine, propylenediamine, n-butylamine, butylenediamine, n-hexylamine, hexamethylenediamine, ammonia water, hexamethyleneimine, piperidine, and piperazine;
preferably, in step (c), a molar ratio of the silicon source II in terms of SiO₂ to the aluminum source II in terms of Al₂O₃ to the water to the template to the organic matter III is 1 : (0.0033-0.010) : (8-20) : (0.1-0.3) : (0.01-0.10), and a ratio of the mass of the mixture B to the total mass of the silicon source II, the aluminum source II, the water, the template and the organic matter III is (0.05-0.2) : 1.

9. A catalyst for producing ethylbenzene by alkylation of benzene, **characterized in that** the catalyst comprises the molecular sieve according to any one of claims 1 to 4 or the molecular sieve prepared by the method according to any one of claims 5 to 9, and optionally a binder such as alumina and/or silica.

10. A method for preparing a catalyst for producing ethylbenzene by alkylation of benzene, comprising:
(e) mixing at least one of the molecular sieve according to any one of claims 1 to 4 and the molecular sieve prepared by the method according to any one of claims 5 to 9, a binder and a polymer mixture, and then molding the resultant mixture, followed by drying, calcining, ammonium exchanging, steam treating, and acid washing, to obtain the catalyst.

11. The method according to claim 10, **characterized in that** the binder in step (e) is an alumina and/or a silica; and/or the polymer mixture in step (e) is a mixture of starch and a surfactant, wherein the starch accounts for 55 to 70 % by mass of the polymer mixture;
preferably, in step (e), a mass ratio of the molecular sieve E to the binder to the polymer mixture is 1 : (0.1-0.4) : (0.03-0.2); and
preferably, in step (e), conditions for the drying include a temperature of 100-200 °C and a period of time of 5-12 hours; conditions for the calcining include a temperature of 500-600 °C and a period of time of 4-10 hours; and conditions for the steam treating include a saturated water vapor atmosphere, a temperature of 300-450 °C and a period of time of 4-10 hours.

12. A method for producing ethylbenzene by alkylation of benzene, **characterized in that** reaction raw materials, benzene and ethylene feedstocks, are contacted with the catalyst according to claim 9 or the catalyst prepared by the method according to any one of claims 10-11 to form a reaction effluent comprising ethylbenzene.

13. The method according to claim 12, **characterized in that** the ethylene feedstock has an ethylene mass concentration of 10 to 100 %; and/or reaction conditions include a reaction temperature of 320 to 380 °C, a reaction pressure of 0.5 to 2.6 MPa, an ethylene mass space velocity of 0.2 to 2.5 h⁻¹, and a molar ratio of benzene to ethylene of 4.0 to 7.0.
